# EUROPEAN PATENT APPLICATION

(11) **EP 2 759 259 A2**
(43) Date of publication of application: **30.07.2014**
(21) Application number: 13186049.6
(22) Date of filing: 26.09.2013
(51) Int. Cl.: A61B 5/024, A61B 5/11, A61B 5/16

(54) **Apparatus and method for measuring stress based on a behavior of a user**

(30) Priority: 24.01.2013 KR 20130008097
(71) Applicant: Samsung Electronics Co., Ltd, Gyeonggi-do 443-742 (KR)
(72) Inventor: Kim, Youn Ho, Yongin-si 446-712 Gyeonggi-do (KR); Bae, Sang Kon, Yongin-si 446-712 Gyeonggi-do (KR); Shin, Kun Soo, Yongin-si 446-712 Gyeonggi-do (KR)
(74) Representative: Grünecker, Kinkeldey, Stockmair & Schwanhäusser

(57) **Abstract**

Provided is an apparatus and method for measuring a stress based on a motion of a user. A stress measurement apparatus may include motion analyzing unit configured to analyze a motion of a user, a stress measurement determining unit configured to determine whether to measure a stress level of the user based on the motion of the user, and a stress assessment unit configured to assess the stress of the user when the stress of the user is determined to be measured.

## Description

### 1. Field

The following description relates to a stress measurement apparatus and method including an apparatus and method for determining whether to measure stress based on a motion of a user, and measuring the stress based on a heart rate of the user.

### 2. Description of Related Art

With continuous interest in the field of medical healthcare and wellbeing, recent research has indicated that various diseases occur due to negative lifestyles. For example, stress that occurs on a daily basis is gradually increasing the levels of mental and psychological diseases.

A variety of methods are typically used to accurately measure stress. However, conventional stress measurement methods are capable of measuring stress only when a user maintains a motionless state for about three to five minutes. Accordingly, the conventional stress measurement methods are significantly inconvenient to the user.

Further, in order to diagnose and treat psychological diseases, in addition to measurement of stress, there is a need to analyze the causes of stress. For example, an analysis on the causes that may increase or decrease stress may be useful for several different diagnosis and treatment purposes.

### SUMMERY

In a general aspect, there is provided a stress measurement apparatus including: a motion analyzing unit configured to analyze a motion of a user; a stress measurement determining unit configured to determine whether to measure stress of the user based on the motion of the user; and a stress assessment unit configured to assess the stress of the user in response to the stress measurement determining unit determining that the stress of the user is to be measured.

The stress assessment unit may be further configured to assess the stress of the user based on a heart rate of the user.

The motion analyzing unit may include any one or any combination of an acceleration sensor, an optical sensor, and a half-cell potential sensor,

The stress measurement determining unit may be configured to determine to measure the stress of the user in response to the motion of the user being maintained at less than or equal to a threshold for a period of time after a standby time has elapsed.

The stress assessment unit may be further configured to perform a spectrum analysis on a peak interval of the heart rate and to extract a stress level based on an activation level of a sympathetic nerve.

The stress assessment unit may be further configured to perform the spectrum analysis on the peak level and determine the stress level based on a power ratio between an activation level of a parasympathetic nerve and the activation level of the sympathetic nerve.

The stress measurement apparatus may further include an event detection determining unit configured to determine whether to detect an event occurring around the user.

The event detection determining unit may be further configured to detect the event as the event associated with a cause of the stress in response to a stress level increasing from a threshold level to a peak level and to detect the event as the event associated with a stress solution in response to the stress level decreasing from the peak level to the threshold level.

The event detection determining unit may be further configured to determine as a cause of the stress any one or any combination of a change in a sound, a change in an image, a change in light, and a change in temperature occurring around the user.

The stress measurement apparatus may further include an output unit configured to output any one or any combination of a stress level of the user and a cause of the stress.

In another general aspect, there is provided a stress measurement method including: analyzing a motion of a user; determining whether to measure stress of the user based on the motion of the user; and assessing the stress of the user in response to a result of the determining being that the stress of the user is to be measured.

The analyzing may include analyzing the motion of the user using any one or any combination of an acceleration sensor, an optical sensor, and a half-cell potential sensor.

The determining may include determining to measure the stress of the user in response to a motion of the user being maintained at less than or equal to a threshold for a period of time after a standby time has elapsed.

The assessing may include assessing the stress based on a heart rate of the user, performing a spectrum analysis on a peak interval of the heart rate, and extracting a stress level based on an activation level of a sympathetic nerve.

The assessing may include performing the spectrum analysis on the peak level and determining the stress level based on a power ratio between an activation level of a parasympathetic nerve and the activation level of the sympathetic nerve.

The method may further include determining whether to detect an event occurring around the user.

The determining whether to detect the event occurring around the user may include detecting the event as the event associated with a cause of the stress in response to a stress level increasing from a threshold level to a peak level, and detecting the event as the event associated with a stress solution in response to the stress level decreasing from the peak level to the threshold level.

The determining whether to detect the event occurring around the user may include determining as a cause of the stress any one or any combination of a change in a sound, a change in an image, a change in light, and a change in temperature occurring around the user.

The method may further include outputting any one or any combination of a stress level of the user and a cause of the stress.

In another general aspect, there is provided a non-transitory computer-readable storage medium storing a program for controlling a computer to perform the method.

### BRIEF DESCRIPTION OF THE DRAWINGS

FIG. 1 is a diagram illustrating an example of an operation of a stress measurement apparatus.
FIG. 2 is a diagram illustrating an example of a stress measurement apparatus.
FIG. 3 is a diagram illustrating another example of a stress measurement apparatus.
FIG. 4 is a graph illustrating an example of a process of determining whether to measure stress based on a motion of a user.
FIG. 5 is a graph illustrating an example of a process of determining an R-R interval (RRI) based on a heart rate of a user.
FIG. 6 is a graph illustrating an example of a process of assessing stress.
FIG. 7 is a graph illustrating an example of a process of analyzing a cause of stress.
FIG. 8 is a flowchart illustrating an example of a stress measurement method.

### DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art. Also, descriptions of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

The features described herein may be embodied in different forms, and are not to be construed as being limited to the examples described herein. Rather, the examples described herein have been provided so that this disclosure will be thorough and complete, and will convey the full scope of the disclosure to one of ordinary skill in the art.

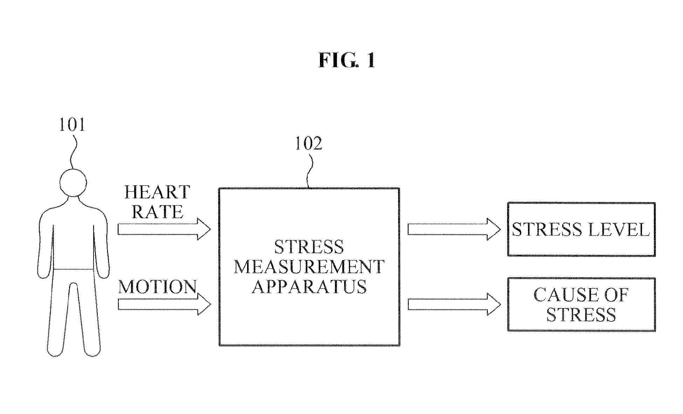

FIG. 1 is a diagram illustrating an example of an operation of a stress measurement apparatus 102.

Referring to FIG. 1, the stress measurement apparatus 102 is configured to determine whether to measure stress based on a motion of a user 101. When a user's 101 motion is less than or equal to a threshold motion that is maintained during a predetermined period of time, the stress measurement apparatus 102 determines that the stress of the user 101 is to be measured.

When it is determined that the stress of the user 101 is to be measured, the stress measurement apparatus 102 measures a stress level of the user 101 based on the user's 101 heart rate. When a stress level of the user 101 exceeds a threshold stress, the stress measurement apparatus 102 detects the cause of stress.

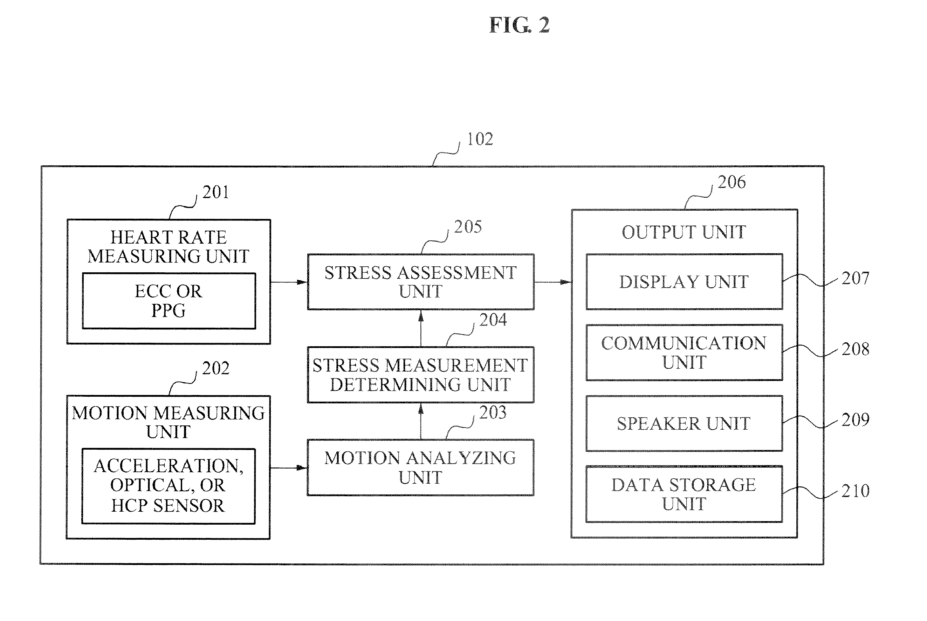

FIG. 2 illustrates an example of the stress measurement apparatus 102.

Referring to FIG. 2, the stress measurement apparatus 102 includes a heart rate measuring unit 201, a motion measuring unit 202, a motion analyzing unit 203, a stress measurement determining unit 204, and a stress assessment unit 205. In an example, the stress measurement apparatus 102 may include an output unit 206. The output unit 206 includes a display unit 207, a communication unit 208, a speaker unit 209, and a data storage unit 210.

In this example, the stress measurement apparatus 102 includes a hear rate measuring unit 201. The heart rate measuring unit 201 is configured to measure a heart rate of a user. For example, the heart rate measuring unit 201 measures a heart rate of the user using an electrocardiogram (ECG) measurement circuit, a photo-plethysmography (PPG) measurement circuit, a pressure sensor, or any other heart rate or biosignal measuring unit.

In this example, the stress measurement apparatus 102 also includes a motion measuring unit 202. The motion measuring unit 202 is configured to analyze the motion of the user using an acceleration sensor, an optical sensor, a half-cell potential (HCP) sensor, or any other motion measuring unit.

In this example, the stress measurement apparatus 102 also includes a motion analyzing unit 203. The motion analyzing unit 203 is configured to analyze the measured motion of the user, For example, the motion analyzing unit 203 analyzes a change in amplitude of a motion signal. Alternatively, the motion analyzing unit 203 may analyze vector magnitudes of X, Y, and Z axes from an acceleration signal that is output through the acceleration sensor.

In this example, the stress measurement apparatus 102 also includes a stress measurement determining unit 204. The stress measurement determining unit 204 is configured to determine whether to measure the stress of a user based on a result of analyzing the motion of the user. For example, when the motion of the user is less than or equal to a predetermined threshold motion that is maintained for a predetermined period of time, the stress measurement determining unit 204 determines to measure the stress of the user. The stress measurement determining unit 204 may perform the determining after a measurement standby time is elapsed.

In this example, the stress measurement apparatus 102 also includes a stress assessment unit 205. When the stress measurement determining unit 204 determines that the stress of the user is to be measured, the stress assessment unit 205 assesses the stress using a heart rate of the user. For example, the stress assessment unit 205 performs a spectrum analysis on a peak interval of the heart rate of the user, and extracts a stress level based on an activation level of a sympathetic nerve. The stress assessment unit 205 performs spectrum analysis on the peak interval and determines stress level using a power ratio between a parasympathetic nerve and the sympathetic nerve.

In an example, the stress measurement apparatus 102 may also include an output unit 206. The output unit 206 is configured to output a stress level assessed by the stress assessment unit 205. The display unit 207 is configured to visually display the output stress level. The communication unit 208 is configured to transfer the output stress level to an external communication apparatus in a wired or wireless manner, The speaker unit 209 is configured to audibly indicate the output stress level. The data storage unit 210 is configured to store the output stress level, and to provide the stored stress level if necessary.

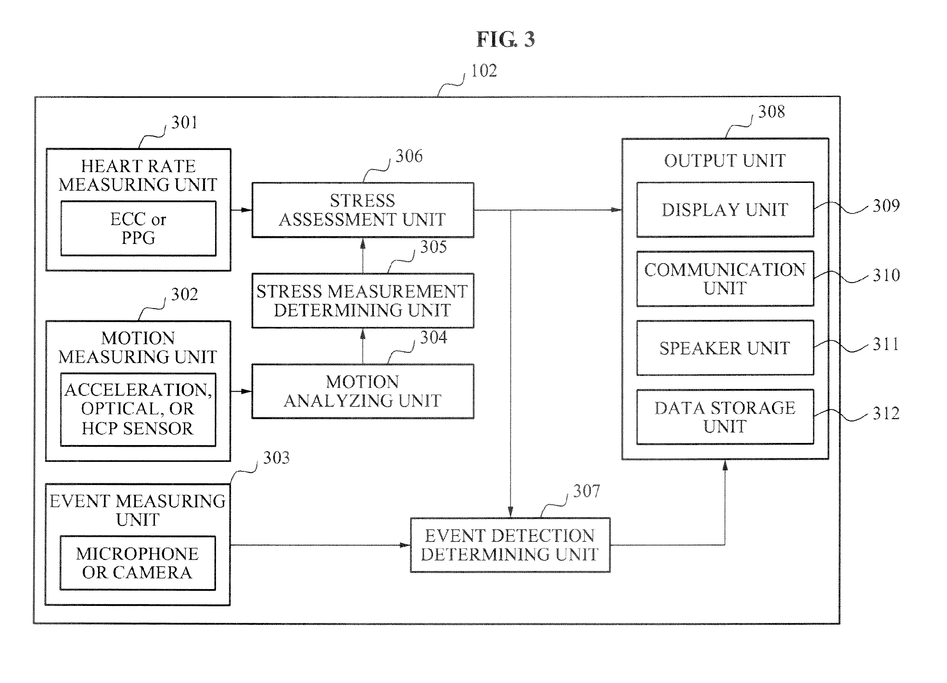

FIG. 3 illustrates another example of the stress measurement apparatus, 102.

Referring to FIG. 3, the stress measurement apparatus, 102 includes a heart rate measuring unit 301, a motion measuring unit 302, an event measuring unit 303, a motion analyzing unit 304, a stress measurement determining unit 305, a stress assessment unit 306, and an event detection determining unit 307. In an example, the stress measurement apparatus 102 may further include an output unit 308. The output unit 308 includes a display unit 309, a communication unit 310, a speaker unit 311 and a data storage unit 312. The stress measurement apparatus 102 of FIG. 3 further includes the event measuring unit 303 and the event detection determining unit 307, in addition to the units previously described with reference to the stress measurement apparatus 102 of FIG. 2.

The description provided for the units similarly described with reference to FIG. 2, such as the heart rate measuring unit 301, motion measuring unit 302, motion analyzing unit 305, stress measurement determining unit 305, and stress assessment unit 306, are also applicable with respect to FIG. 3. In this example, the event detection unit 307 receives the output stress level from the stress assessment unit 306 for determining whether to detect an event occurring around the user.

In this example, the stress measurement apparatus 102 includes an event measuring unit 303. In response to a request from the event detection determining unit 307, the event measuring unit 303 measures an event, such as, a change in a sound, a change in an image, a change in light, or a change in temperature. For example, the event measuring unit 303 may measure an event which occurs around a user using a microphone, a camera, a temperature sensor, an optical sensor, and the like.

In this example, the stress measurement apparatus 102 also includes an event detection determining unit 307. The event detection determining unit 307 is configured too determine whether to detect an event occurring around the user while assessing the stress. For example, when a stress level increases from a threshold level to a peak level, the event detection determining unit 307 detects the event associated with the cause of stress. This detection may be performed during the interval in which the stress level increases. On the contrary, when the stress level decreases from the peak level to the threshold level, the event detection determining unit 307 detects the event associated with the stress solution during the interval in which the stress level decreases.

In this example, the event detection determining unit 307 is configured to detect, as the cause of stress, a change in a sound, a change in an image, a change in light, or a change in temperature that occur around the user. Any other event changes that may be sensed, such as olfactory, kinetic, tactile, and the like, may also be detected.

In this example, the output unit 308 is configured to output the stress level assessed by the stress assessment unit 306 and the event that is the cause of stress as detected by the event detection determining unit 307. The display unit 309 is configured to visually display the output stress level and cause of stress. The communication unit 310 is configured to transfer the output stress level and cause of stress to an external communication apparatus in a wired or wireless manner. The speaker unit 311 is configured to audibly indicate the output stress level and cause of stress. The data storage unit 312 is configured to store the output stress level and cause of stress, and provide the stored stress level and cause of stress if necessary.

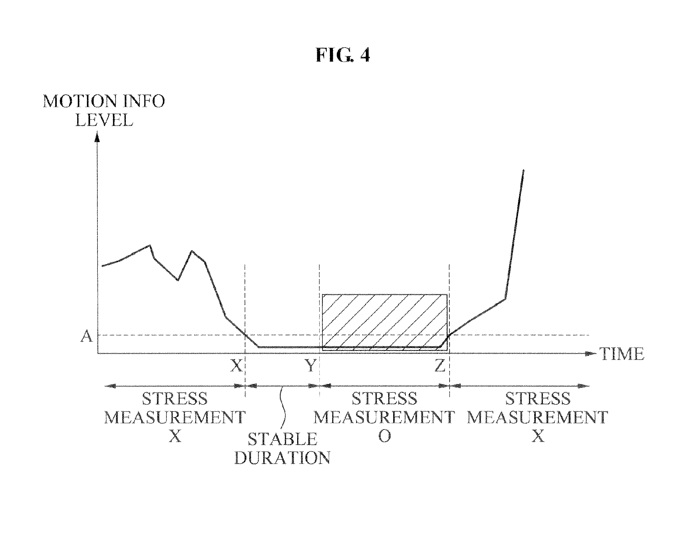

FIG. 4 illustrates an example of a process of determining whether to measure the stress of a user based on a motion of a user.

Referring to FIG. 4, a threshold motion of the motion information is designated by the line A. In this example, during the time interval from "0" to X, the motion level of a user is equal to or more than the threshold level A. Thus, the stress measurement apparatus determines not to assess the stress level of the user.

During the time interval from X to Y, the motion level of the user is less than or equal to the threshold level A. However, user motion was present in the time interval from "0" to X, thus the user may still be in an excited state. Accordingly, in this example, even though the motion level of the user is less than or equal to the threshold level A, the stress measurement apparatus waits for a period of time until the user becomes stabilized. Therefore, in this example, the time interval from X to Y may be set to a measurement standby time.

During the time interval from Y to Z, the motion level of the user is less than or equal to the threshold level A. Accordingly, the user is in a stable condition and the stress measurement apparatus may determine to assess the stress level of the user. During the time interval after Z, the user's motion exceeds the threshold level A, thus the stress measurement apparatus may determine to not assess the stress level of the user.

In this example, when the motion level of the user is less than or equal to a threshold level A of motion, and the motion is maintained for a predetermined period of time, the stress measurement apparatus may determine that the stress level of the user is to be measured.

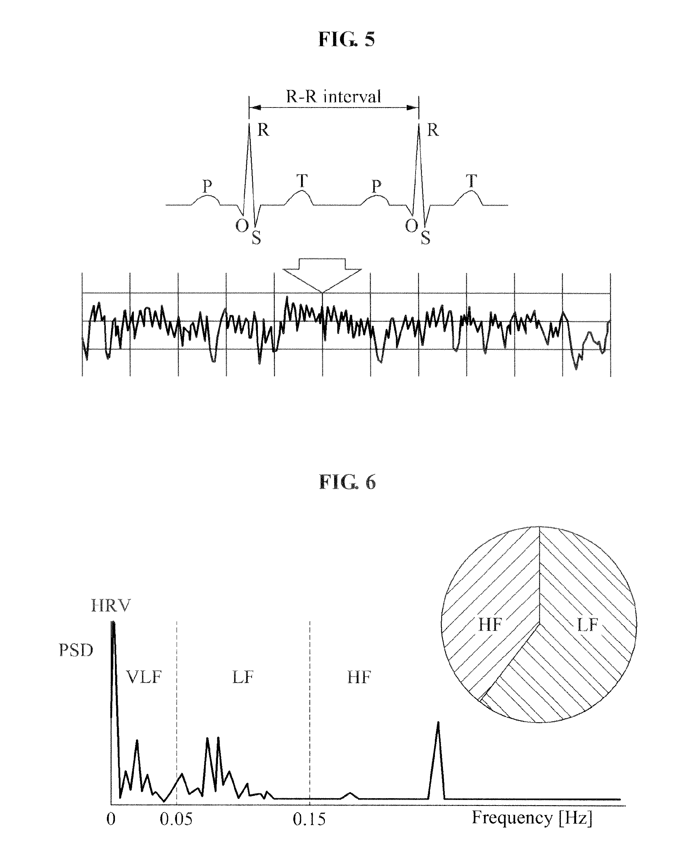

FIG. 5 illustrates an example of a process of determining an R-R interval (RRI) based on the heart rate of the user.

In this example, the stress measurement apparatus collects a change in an R peak interval (RRI) using an ECG of a user during a predetermined period of time. The graph shown on the bottom of FIG. 5 shows a change in the RRI that is acquired during the predetermined period of time. The change in the RRI varies based the activity of the autonomic nervous system. The autonomic nervous system includes a sympathetic nerve and a parasympathetic nerve.

FIG. 6 illustrates an example of a process of assessing stress. In this example, the stress measurement apparatus analyzes a frequency spectrum of the change in the RRI. By analyzing the frequency spectrum, the stress measurement apparatus may assess a stress level of a user.

It should be appreciated that when a sympathetic nerve is stimulated a reaction time is generally delayed about five seconds compared to when a parasympathetic nerve is stimulated. Accordingly, simulation to the parasympathetic nerve corresponds to a rapid change in the RRI.

As a result of analyzing the frequency spectrum, a low frequency band is associated with simulating of the sympathetic nerve of the user, and a high frequency band is associated with simulating of the parasympathetic nerve of the user. The sympathetic nerve is activated when the user is in an excited or nervous sate, and the parasympathetic nerve is activated when the user is in a stable state. In this example, the low frequency band and the high frequency band are classified based on about 0.1 Hz.

In this example, the stress measurement apparatus assesses the stress level of the user using a ratio between an integral value of low frequency (LF) and an integral value of high frequency (HF) that are obtained as a result of analyzing the frequency spectrum of the change in the RRI. This ratio may be referred to as a power ratio. In this example, the ratio between the integral value of LF and the integral value of HF indicates the activation level of the sympathetic nerve. Based on the level at which the sympathetic nerve is activated, the stress measurement apparatus determines the level of stress of the user. That is, in this example, when the user is in a normal state, a ratio between the integral value of LF and the integral value of HF is about 6:4. When the user is in a stress state, the ratio between the integral value of LF and the integral value of HF is greater.

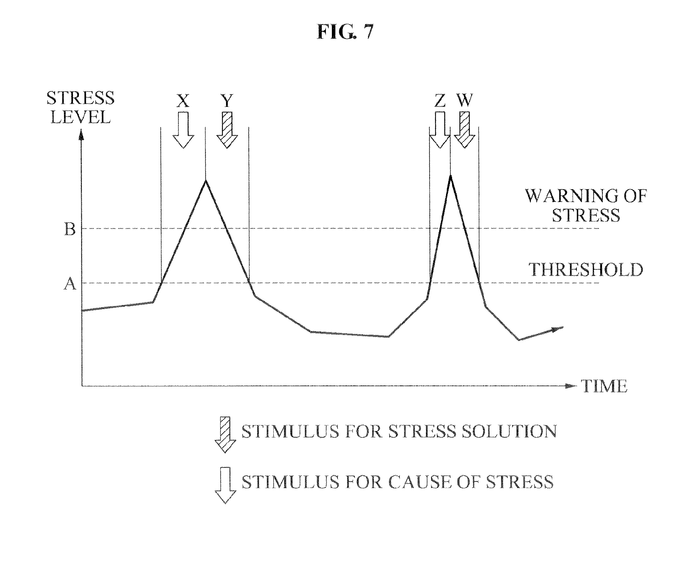

FIG. 7 illustrates an example of a process of analyzing a cause of stress.

Referring to FIG. 7, in time intervals X and Z, it can be seen that a stress level of a user increases to be greater than a threshold stress level A. This example indicates that a negative event is affecting the user and a cause of increasing stress has stimulated the user. In time intervals Y and W, it can be seen that the stress level of the user decreases. This example indicates that a positive event is affecting the user and a cause of decreasing stress has stimulated the user.

Accordingly, during the time interval from a user exceeding a threshold stress level and up to a peak stress level, such as time intervals X and Z, the stress measurement apparatus detects an event corresponding to a stimulus for cause of stress. On the contrary, during the time interval from the peak stress level to the threshold stress level, such as time intervals Y and W, the stress measurement apparatus detects an event corresponding to a stimulus for stress solution.

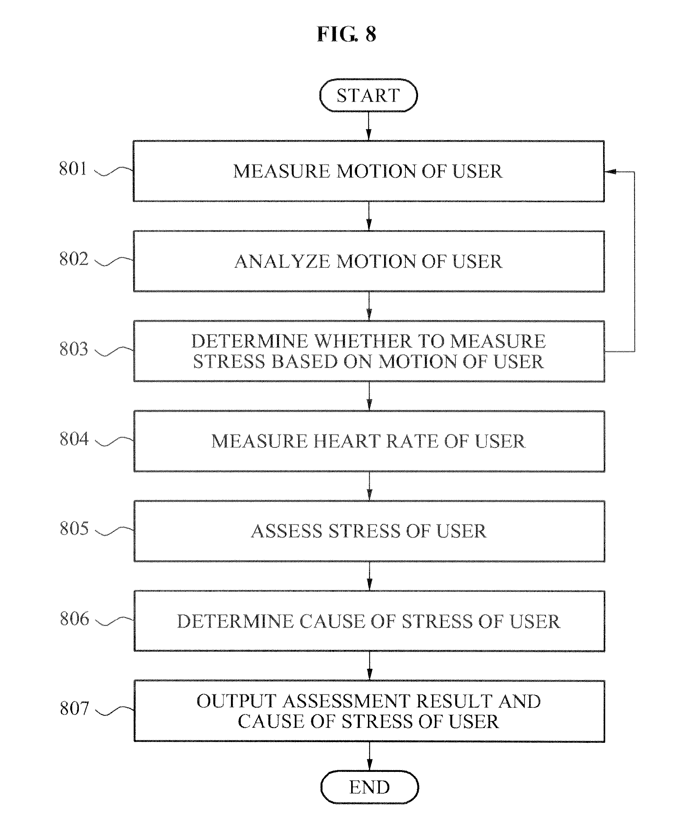

FIG. 8 illustrates an example of a stress measurement method.

In operation 801, a stress measurement apparatus measures a motion of a user. For example, the stress measurement apparatus analyzes the motion of the user using at least one of an acceleration sensor, an optical sensor, an HCP sensor, or other motion measuring unit.

In operation 802, the stress measurement apparatus analyzes the measured motion of the user. For example, the stress measurement apparatus analyzes a change in the amplitude of a motion signal. Alternatively, the stress measurement apparatus may analyze vector magnitude of X, Y, and Z axes from an acceleration signal that is output through the acceleration sensor.

In operation 803, the stress measurement apparatus determines whether to measure stress of the user based on a result of analyzing the motion of the user. For example, when the motion of the user is less than or equal to a predetermined threshold motion, and maintained as such during a predetermined period of time after a measurement standby time is elapsed, the stress measurement apparatus determines to measure the stress of the user.

When the motion of the user is greater than the threshold motion, the stress measurement apparatus measures the motion of the user in operation 801 again.

In operation 804, the stress measurement apparatus measures a heart rate of the user. For example, the stress measurement apparatus measures the heart rate of the user using an ECG measurement circuit, a PPG measurement circuit, a pressure sensor, or other biosignal measuring unit.

In operation 805, the stress measurement apparatus assesses the stress using the heart rate of the user. For example, the stress measurement apparatus performs a spectrum analysis on a peak interval of the heart rate of the user. The stress measurement apparatus extracts or determines a stress level based on an activation level of a sympathetic nerve. The stress measurement apparatus performs the spectrum analysis on the peak level and determine the stress level using a power ratio between the parasympathetic nerve and the sympathetic nerve.

In operation 806, the stress measurement apparatus determines whether to detect an event that occurs around the user while assessing the stress. For example, when a stress level increases from a threshold level to a peak level, the stress measurement apparatus detects an event associated with a cause of stress during the interval in which the stress level increases. On the contrary, when the stress level decreases from the peak level to the threshold level, the stress measurement apparatus detects an event associated with a stress solution during the interval in which the stress level decreases.

In this example, the stress measurement apparatus measures a change in a sound, a change in an image, a change in light, a change in temperature, or any other detectable changes related to an event which occurs around a user. These changes may be detected using a microphone, a camera, a temperature sensor, an optical sensor, and the like.

In operation 807, the stress measurement apparatus outputs the assessed stress level and the event corresponding to the cause of stress. In an example, the stress measurement apparatus visually displays the output stress level and cause of stress. The stress measurement apparatus may transfer the output stress level and cause of stress to an external communication apparatus in a wired or wireless manner. The stress measurement apparatus may audibly indicate the output stress level and cause of stress. The stress measurement apparatus may store the output stress level and cause of stress, and may provide the stored stress level and cause of stress if necessary.

According to various examples herein, when a user maintains a motionless state during a predetermined period of time after the user has arbitrarily moved, a stress measurement apparatus may autonomously measure a stress level of the user. In other words, without a user being asked to artificially maintain a stable state, the stress measurement apparatus analyzes a motion of the user and determines whether to measure the stress level of the user. Accordingly, using the stress measurement apparatus, a user may verify or recognize a stress occurring during their regular lifestyle without the need to artificially control the motion of the user.

According to various examples herein, the stress measurement apparatus determines a cause that increases or decreases stress of a user based on a change in a stress level. For example, the stress measurement apparatus provides causes that affect a stress level of the user based on a sound, an image, a light, a heat, or other even changes that occur around the user. This enables a user to recognize the activities or events that cause an increase in stress, and allows the user to prevent or limit such activities in order to avoid stress related diseases.

It should be appreciated that while the disclosure describes analyzing a motion of a user for determining whether to measure stress of the user, the determining may be performed by analyzing any other behavior of a user. In an example, determining whether to measure stress of a user may be performed by analyzing brain activity, digestive activity, or side effects of disease having behavioral consequences to the user. Additionally, a threshold level of such behavior may be set for determining whether to detect stress of the user.

The stress measurement apparatus 102 and all units described above may be implemented using one or more hardware components, or a combination of one or more hardware components and one or more software components. A hardware component may be, for example, a physical device that physically performs one or more operations, but is not limited thereto. Examples of hardware components include controllers, microphones, amplifiers, low-pass filters, high-pass filters, band-pass filters, analog-to-digital converters, digital-to-analog converters, and processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

Software or instructions for controlling a processing device, such as those described in FIG. 8, to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

For example, the software or instructions and any associated data, data files, and data structures may be recorded, stored, or fixed in one or more non-transitory computer-readable storage media. A non-transitory computer-readable storage medium may be any data storage device that is capable of storing the software or instructions and any associated data, data files, and data structures so that they can be read by a computer system or processing device. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magnetooptical data storage devices, optical data storage devices, hard disks, solid-state disks, or any other non-transitory computer-readable storage medium known to one of ordinary skill in the art.

Functional programs, codes, and code segments for implementing the examples disclosed herein can be easily constructed by a programmer skilled in the art to which the examples pertain based on the drawings and their corresponding descriptions as provided herein.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the disclosure is defined not by the detailed description, but by the claims.

## Claims

1. A stress measurement apparatus, comprising:
a motion analyzing unit configured to analyze a motion of a user;
a stress measurement determining unit configured to determine whether to measure stress of the user based on the motion of the user; and
a stress assessment unit configured to assess the stress of the user in response to the stress measurement determining unit determining that the stress of the user is to be measured.

2. The stress measurement apparatus of claim 1, wherein the stress assessment unit is further configured to assess the stress of the user based on a heart rate of the user.

3. The stress measurement apparatus of claim 1 or 2, wherein the motion analyzing unit comprises any one or any combination of an acceleration sensor, an optical sensor, and a half-cell potential sensor.

4. The stress measurement apparatus of one of claims 1 to 3, wherein the stress measurement determining unit is configured to determine to measure the stress of the user in response to the motion of the user being maintained at less than or equal to a threshold for a period of time after a standby time has elapsed.

5. The stress measurement apparatus of claim 2, wherein the stress assessment unit is further configured to perform a spectrum analysis on a peak interval of the heart rate and to extract a stress level based on an activation level of a sympathetic nerve.

6. The stress measurement apparatus of claim 5, wherein the stress assessment unit is further configured to perform the spectrum analysis on the peak level and determine the stress level based on a power ratio between an activation level of a parasympathetic nerve and the activation level of the sympathetic nerve.

7. The stress measurement apparatus of one of claims 1 to 6, further comprising an event detection determining unit configured to determine whether to detect an event occurring around the user.

8. The stress measurement apparatus of claim 7, wherein the event detection determining unit is further configured to detect the event as the event associated with a cause of the stress in response to a stress level increasing from a threshold level to a peak level and to detect the event as the event associated with a stress solution in response to the stress level decreasing from the peak level to the threshold level.

9. The stress measurement apparatus of claim 7, wherein the event detection determining unit is further configured to determine as a cause of the stress any one or any combination of a change in a sound, a change in an image, a change in light, and a change in temperature occurring around the user.

10. The stress measurement apparatus of one of claims 1 to 9, further comprising an output unit configured to output any one or any combination of a stress level of the user and a cause of the stress.

11. A stress measurement method, comprising:
analyzing a motion of a user;
determining whether to measure stress of the user based on the motion of the user; and
assessing the stress of the user in response to a result of the determining being that the stress of the user is to be measured.

12. The method of claim 11 adapted to operate the stress measurement apparatus of one of claims 1 to 10.

13. A non-transitory computer-readable storage medium storing a program for controlling a computer to perform the method of claim 12.
